# EUROPEAN PATENT SPECIFICATION

(11) **EP 1 312 315 B1**
(45) Date of publication and mention of the grant of the patent: **03.08.2005**
(21) Application number: 02356012.1
(22) Date of filing: 25.01.2002
(51) Int. Cl.: A61B 17/34, A61F 2/10, A61B 10/00

(54) **Hair transplant device**
Haartransplantationsvorrichtung
Dispositif de greffe capillaire

(30) Priority: 17.11.2001 KR 2001071619
(43) Date of publication of application: 21.05.2003
(73) Proprietor: HB Medicals Corporation, Seoul 121-815 (KR); Lee, Hoon Bum, Seoul, 135-280 (KR)
(72) Inventor: Lee, Hoon Bum, Kangnam-Gu, Seoul 135-280 (KR)
(74) Representative: Moinas, Michel

(56) References cited:
- WO-A-01/65996
- US-A- 5 611 811
- US-B1- 6 248 081

## Description

### BACKGROUND OF THE INVENTION

The present invention relates to a hair transplant device, and in particular to the device capable of harvesting a hair graft from a hairy area of a scalp and then implanting it into a bald area thereof in series.

Hair of a scalp is one of important factors for expressing a human appearance. Bald areas of the scalp may increase with age, and the number of hair may decrease due to scars of the head skin resulted from operations or diseases.

Conventional methods to solve such problems, currently used are one by a medicinal therapy and one by an operation. A representative example of the medicinal therapy is to apply certain topical medicine such as "ROGAINE" (trademark of Upjohn) or to take certain medicine such as "PROPECIA" (trademark of Merck). The former should be applied to bald areas or hair falling-out areas (hereinafter, referring to as "bald area") at the specific time about two times a day. However, it is very high at the price and very viscous substance, whereas the efficacy of the medicine is not more than about 5%. Furthermore, when not applied regularly, it is not efficacious. Meanwhile, the latter shows a little higher efficacy than "ROGAINE", but it should be taken everyday and causes harmful side effects; for example, it has been reported about 15% of men who took them suffered from impotentia generandi and women cannot even take them.

Accordingly, as an essential method, the operative method for directly transplanting some hairs from hairy areas into bald areas has been used. This method allows a human appearance to become beautiful by growing hairs, stemming from areas not showing depilation symptoms (hereinafter, referred to as "hairy area"). A conventional procedure of such a transplantation operation can be briefly described as the following: cutting hairs of a hairy area required for operation, harvesting a piece of scalp of the hairy area by about 1 cm × 10 cm size to obtain a transplanting skin (so called, "donor scalp"), pulling and suturing a remaining portion after harvest, separating each of hairs (including hair roots) from the donor scalp, making incisions in a bald area, and then implanting the separated hairs into the incisions one by one.

These transplanting method can be classified into three types according to an amount of hairs to be transplanted at one time: micro-graft method of transplanting one hair follicles, mini-graft method of transplanting two or three hairs follicles, and composite-graft method of transplanting a tissue containing more than three hairs. In the case of composite-graft method, it is impossible to transplant the desirable number of hairs and further the falling-out of hair can easily occur after transplantation; therefore, the micro-graft method and the mini-graft method are mainly used. However, in the case of the two methods, a possibility of injuring the hair follicles is very high and it takes much time to separate the hair follicles, thereby the operation time is generally long. In the case of the micro-graft method, it is difficult to pick hairs by two or three units.

In the meantime, devices used in the hair transplanting operation include one for cutting a scalp of hairy areas, one for separating the cut scalp to hair units for transplantation including at least one hair, one for making an incision in bald areas, one for implanting the separated hair graft into the incision, and so on. Herein, the step of harvesting the hair units and the step of implanting are very elaborate and time-consuming; therefore, in order to address these problems, various advanced devises have been developed.

For example, U.S. Patent No. 5,611,811, titled "Micro And Mini Hair Transplant Device," discloses a device comprising (1) a part for puncturing the scalp, (2) a part for containing the hair grafts to be transplanted, (3) a part for ejecting the hair grafts from the containing means, (4) a part for actuating the ejecting means, and (4) a part for delivering the hair grafts into the transplant site. More particularly, this document discloses a device in conformity with the preamble of claim 1.

U.S. Patent No. 5,693,064, titled "Dermal Punch For Hair Transplantation And Methods," discloses a dermal punch comprising a concentric shaft having a proximal end, a distal end, and an axis extending therebetween.

U.S. Patent No. 5,817,120, titled "Hair implanting instrument," discloses a device comprising (1) an elongate housing adapted to be manipulated by a surgeon during implanting of the hair grafts, (2) a cutting device affixed to the first end of the elongate housing, and (3) an implanting member disposed axially movably within the throughbore of the elongate housing.

U.S. Patent No. 5,873,888, titled "Surgical Instrument For The Transplantation Of Self-grown Hair," discloses a device comprising (1) a cartridge adapted to hold at least two micrografts, having an elongated body and a lid, and (2) a body member adapted to be grasped by a surgeon, having a barrel portion, a scalpel blade, indexing means and a plunger mechanism.

The devices disclosed in these patents were based upon the concept that the step of harvesting one, two or three hairs units from a hairy area and the step of implanting these hair units into incisions in a bald area are executed separately, and thus were developed to execute efficiently one of both steps.

However, in the case that both the hair unit-harvesting step and the hair unit-implanting step are executed separately as in the above patents, operation itself is very time-consuming and elaborate; therefore, a patient suffers pain and it costs a great deal. Furthermore, as described above, for harvest of the hair units, it is necessary to cut off the scalp for transplantation by about 1 cm × 10 cm and then suture the remaining portion after cut-off; however, the suture portion cracks to about 5 to 7 mm at the width after an elapse of about two months, and thereby new scar is left without hairs.

It would therefore be desirable to provide devices capable of executing the two steps in series, i.e., both the step of harvesting the hair units from a bald area and the step of implanting them into a hairy area. In addition, it would be more desirable to provide devices capable of pinching only a small hair graft without cutting off a relatively large scalp, and thereby leaving no scar and reducing patients' pain.

### SUMMARY OF THE INVENTION

The present invention addresses the foregoing need by providing a hair transplant device that can harvest a hair graft from hairy areas in a scalp and then implant it into bald areas in series, which comprises:
first cutting member affixed to a front part of the present device and having an elongate and hollow shape, wherein the first member has a blade of which the surface ("imaginary surface") inclines toward a side of the present member, wherein a hair graft is cut by the blade and then is introduced into the present member;
second cutting member disposed to move forward/backward (or revolve) in the first cutting member and having an elongate and hollow shape, wherein the second cutting member has a blade of which the surface ("imaginary surface") cuts a bottom portion of the hair graft having been introduced into the present device and then meets the blade surface of the first cutting member, as the second cutting member moves forward;
extrusion member disposed to move forward/backward in the second cutting member and having an elongate shape, wherein the extrusion member pushes out the hair graft loaded in the present device via an entrance of the blade surface of the first cutting member, as the extrusion member moves forward; and,
housing forming an outward shape and providing an induction road for movement of the second cutting member and the extrusion member.

The term "hair graft," used in the present specification, means a hair unit containing at least two hairs and preferably two to four hairs. Therefore, it should be noted that the hair transplant device according to the present invention is suitable for macro-graft method.

The hair transplant device according to the present invention allows the below steps to be executed in series:
a step for inserting the first cutting member into a bald area in a scalp, wherein the scalp is cut by the blade of the first cutting member to become the form of a hair graft;
a step for moving the second cutting member forward (or revolving) and cutting a bottom portion of the hair graft, thereby harvesting the hair graft for transplantation,
a step for inserting the first cutting member, in which the hair graft is loaded, into the bald area of the scalp,
a step for moving the second cutting member backward (or revolving) and then moving the extrusion member forward,
a step for withdrawing the first cutting member from the bald area, thereby implanting the hair graft in the bald area.

One of features of the present invention is that the blade surface, forming the entrance of the first cutting member, inclines toward the side of the present device, and at forward movement of the second cutting member, the blade surface thereof meets the blade surface of the first cutting member. The configurations of the first cutting member and the second cutting member are not particularly restricted as long as the above requirements are satisfied, but the cylindrical form is more preferable than any other forms, because a cylindrical incision, left after harvest, can be healed speedily and a cylindrical hair graft maintain stability. Accordingly, in particularly preferred embodiments of the present invention, the blade surfaces of the first and second cutting members is fabricated or treated to have distal ends of the cylindrical members to meet each other at fully forward movement of the second cutting member.

Regarding the first cutting member, since the surface of the blade thereof (imaginary plane) inclines toward one side face of the cylindrical member, the opposite side face of the cylindrical side gets gently bent to extend toward the above side which the blade surface faces, whereby a cross-sectional shape of the blade surface become ellipse or circle. A horizontal axis of the blade surface is generally perpendicular to a central axis of the first cutting member, but the horizontal axis of the blade surface may incline somewhat such that the blade surface can be seen a little from the front sight of the present device. Therefore, when inserted into the hairy area for harvest of the hair graft, the present device is intended to be inserted at the angle where the blade surface faces toward the hairy area.

Regarding the second cutting member, the direction of the blade surface thereof is opposite to the direction of the blade of the first cutting member on the whole. Therefore, when the second cutting member moves forward fully, a distal end of the second cutting member ultimately meets a distal end of the first cutting member. In order to minimize a damage of the hair graft when cutting the bottom portion thereof, which has been introduced into the first cutting member, the blade surface of the second cutting member is preferably sunken. In the case that the horizontal axis of the blade surface inclines a little, as described above, the distal end of the second cutting member inclines from the side of the cylindrical member (the side that the blade surface of the first cutting member faces), to the extent that the distal end of the second cutting member parts from an imaginary extended line of the side of the first cutting member, whereby the distal end of the first cutting member can meet the distal end of the second cutting member at fully forward movement of the latter.

The description so far shows that the second cutting member is disposed in the first cutting member, but the reverse configuration thereof is also possible. In a preferred embodiment of the present invention, a cylindrical second cutting member is disposed in a cylindrical first cutting member, and an outer diameter of the second cutting member is near to an inner diameter of the first cutting member, and the second cutting member can move forward and backward along an inner surface of the first cutting member. Accordingly, when the second cutting member moves backward, an entrance of the blade surface of the first cutting member becomes opened. To the contrary, when the second cutting member moves forward, the entrance of the blade surface of the first cutting member becomes shut.

In the above, described are mainly the configuration that the entrance of the first cutting member becomes shut or opened by forward or backward movement of the second cutting member; however, in any case, opening and shutting of a first cutting member may be executed by revolution of a second cutting member. At one concrete example of such a configuration, a distal end of a first cylindrical cutting member inclines curvilinearly to a central axis of the first cutting member, and a cylindrical second cutting member, disposed in the first cutting member, has the same shape as the cylindrical first cutting member, whereby when each blade surface of both cutting members is in the same position, an entrance into the device becomes opened, and when the second cutting member is revolved, the entrance becomes shut.

The extrusion member works as pushing the hair graft, loaded in the first cutting member, out the device by moving forward. Therefore, in the case that the second cutting member is disposed in the first cutting member, the extrusion is disposed in the second cutting member. To the contrary, in the case that the first cutting member is disposed in the second cutting member, the hair graft is disposed in the first cutting member.

A shape of an distal end of the extrusion member is not particularly restricted, but the distal end shape is preferably tapered (i.e., cut inclined) opposite to the blade surface of the first cutting member. A hair graft to be introduced into the cutting member starts to be cut by a distal end of the blade surface of the first cutting member. As a result, the hair graft loaded in the cutting member has the end thereof inclined opposite to the blade surface of the first cutting member. Therefore, in order to extrude the hair graft with an equivalent pressure to be applied thereto, it is desirable for the distal end of the extrusion member to be tapered opposite to the blade surface, as described above. In any case, the extrusion member may protrude a little from the entrance of the first cutting member at fully forward movement of the extrusion member, whereby assisting in implanting the hair graft into a bald area. In this case, the extrusion member is made of flexible materials such as polyethylene, polybutadiene, and the like, such that the member can bend along a curved path of the cutting member.

The housing comprises a conical front part affixing the first cutting member, and a body providing an induction road for movements of the second cutting member and the extrusion member, of which a shape is not particularly restricted. The conical front part and the body may be in a unitary form or may be in an assembling form. In particular, various configurations are available for the housing, depended upon methods for providing powers for movements of the second cutting member and the extrusion member, which can be briefly classified into a manual type and an automatic type. Therefore, handles or switches for operation of the second cutting member and the extrusion member can have various forms, as will be described below.

As representative examples of the manual type, the below three configurations are provided according to the present.

### Configuration 1

The first cutting member is affixed in the conical front part of the housing;
the second cutting member is supported by a first spring installed in the conical front part, and is connected to a first operating handle disposed at the middle of a body of the housing; and
the extrusion member is supported by a second spring installed in the body, and is connected to a second operating handle disposed at the rear of the body.

Configuration 1 is an essential configuration of the present invention. When the first operating handle connected to the second cutting member is pushed ahead, the blade surface of the second cutting member meets the blade surface of the first cutting member. When a pressure applied to the first operating handle is removed, the second cutting member recedes automatically by an elastic recovering force of the first spring. In the same manner, when the second operating connected to the extrusion member is pushed ahead, the hair graft loaded in the device is pushed out through the entrance of the first cutting member. When a pressure applied to the second operating handle is removed, the extrusion member recedes automatically by an elastic recovering force of the second spring. In any case, the second operating handle may be disposed at the middle of the body by deforming a structure, as in the first operating handle.

### Configuration 2

The first cutting member is affixed in the conical front part of the housing,
the second cutting member is supported by a first spring installed in the conical front part, and is connected to an operating handle disposed at the middle of a body of the housing, wherein a rear portion of the operating handle is extended to a power transmitting member;
the extrusion member is supported by a second spring installed between a connecting axis to the operating handle and a middle portion of the body, and is extended to the power transmitting member; and
the power transmitting member comprises a gear which revolves with its axis affixed in the body, wherein the gear engages with a thread which is formed on the bottom of a rear extension part of the operating handle, and a thread which is formed on the top of a rear extension part of the extrusion member, respectively.

A feature of Configuration 2 is that forward/backward movements of the second cutting member and the extrusion member are controlled by only one operating handle, different from in Configuration 1. This control becomes possible by the fact that the movement directions of the bottom and top threads are opposite to each other due to the gear as the power transmitting member. That is, the movement of the top thread, connected to the second cutting member, is transmitted to the bottom thread connected to the extrusion member at the direction reversed by the gear.

An operation procedure of Configuration 2 is described below in detail. Under the state that both the second cutting member and the extrusion member recede fully ("Rest mode"), the operating handle is at the middle position. Under the state that the second cutting member moves forward and, thereby, its blade surface meets the blade surface of the first cutting member ("Hair-harvesting mode"), the second cutting member moves forward while the operating handle is pushed ahead, which forces the gear to revolve, whereby the extrusion member recedes by backward movement of the bottom thread. Under the state that the hair graft loaded in the device is extruded by forward movement of the extrusion member ("Hair-implanting mode"), the second cutting member recedes while the operating handle is pulled back, which force the gear to revolve conversely, whereby the extrusion member moves forward by forward movement of the bottom thread.

### Configuration 3

the first cutting member is affixed in the conical front part;
the second cutting member is supported by a first spring installed in the conical front part, wherein triangle members are installed on the second cutting member at the position corresponding to a first operating handle disposed on the middle of a body of the housing, wherein a perpendicular face of the first triangle is arranged forwardly;
the extrusion member is supported by a second spring installed in the middle of the body, and is connected to a second operating handle disposed at the rear of the body; and
the first operating handle includes second triangle members, wherein a slope face of the second triangle member is sliding contact with a slope face of the first triangle member.

A feature of Configuration 3 is that both the first operating handle disposed on the side of the body and the second operating handle disposed on the rear of the body are button type.

An operation procedure of Configuration 3 is described below in detail. When the first operating handle is pushed down under the hair-harvesting mode, the second triangle member in the first operating handle descend and, simultaneously, the slope face of the second triangle member compresses the slope face of the first triangle member on the second cutting member. In order to eliminate this compression force, the first triangle member moves forward and, thereby, the second cutting member also moves forward. Operations of other members are identical with those in Configuration 1.

One concrete example of the automatic type is provided below by the present invention.

### Configuration 4

The first cutting member is affixed in the conical front part;
the second cutting member includes an extension part at the rear thereof, wherein an inner surface of the extension part has a thread engaged with a power-transmitting member linked to an electric motor;
the extrusion member includes a thread engaged with the power-transmitting member on an outer surface thereof; and
the power-transmitting member includes a thread engaged with the thread of the extension part, and a thread engaged with the thread of the extrusion member, wherein the two threads of the power-transmitting member are formed at the opposite direction to each other.

A feature of the automatic type-hair transplant device is that both the hair-harvesting mode and the hair-implanting mode can be executed by easy control of the switch, different from in the manual type ones. Power for operating the motor may be provided by first batteries (so called, dry cells) or secondary rechargeable batteries within the body, or by electric cables connected to exterior power supplies. Regarding operation of the power-transmitting member, for example, when the electric motor turns right, the second cutting member moves forward while the extrusion member recedes, and when the electric motor turns left, the cutting member recedes while the extrusion member moves forward.

In a representative configuration of such power-transmitting member, a right directional thread is formed, engaging with the thread of the second cutting member, on the outer surface of the power-transmitting member, whereas a left directional thread is formed, engaging with the thread of the extrusion member, on the inner surface thereof. In any case, a control switch is installed outside the device so as for a surgeon to use it easily; for example, the control switch is installed at the position of the surgeon's feet as in a scaffold form.

Features and advantages of the present invention will appear more clearly on a reading of the following detailed description of each of members illustrating preferred embodiments of the features of the present invention, the description being by way of non-limiting examples only and with reference to the accompanying drawings.

### BRIEF DESCRIPTION OF THE DRAWINGS

FIG. 1 shows a perspective view of the hair transplant device according to a first preferred embodiment of the present invention.

FIGS. 2A and 2B show partially exploded perspective views of the device in FIG 1 during a hair-harvesting mode and a hair-implanting mode, respectively.

FIGS. 3A and 3B show a cross-sectional view, a front view and a side view of front portions of the first cutting members according to preferred embodiments of the present invention, respectively.

FIGS. 4A and 4B show a front view, a cross-sectional view and a side view of a front portion of the second cutting member according to a preferred embodiment of the present invention, respectively.

FIGS. 5A and 5B show partially exploded sectional views of the first and second cutting members according to a modified embodiment of the present invention.

FIGS. 6A and 6B show perspective views of the device according to a modified embodiment of the present invention.

FIGS. 7A - 7C show perspective views of the device according to a second embodiment of the present invention during a hair transplantation procedure.

FIGS. 8A - 8C show perspective views of the device according to a third embodiment of the present invention during a hair transplantation procedure.

FIG. 9 shows a perspective view and a partially exploded view of the device according to a forth embodiment of the present invention, respectively.

FIGS. 10A - 10E show perspective views of a hair transplantation procedure according to the present invention.

### DESCRIPTION OF THE MAIN REFERENCE NUMBERS

100, 101, 102, 103: hair transplant devices
200, 201, 202, 203: first cutting members
300, 301, 302, 303: second cutting members
400, 401, 402, 403: extrusion members
500: housing
610, 620: springs

### DETAILED DESCRIPTION OF THE SPECIFIC EMBODIMENTS

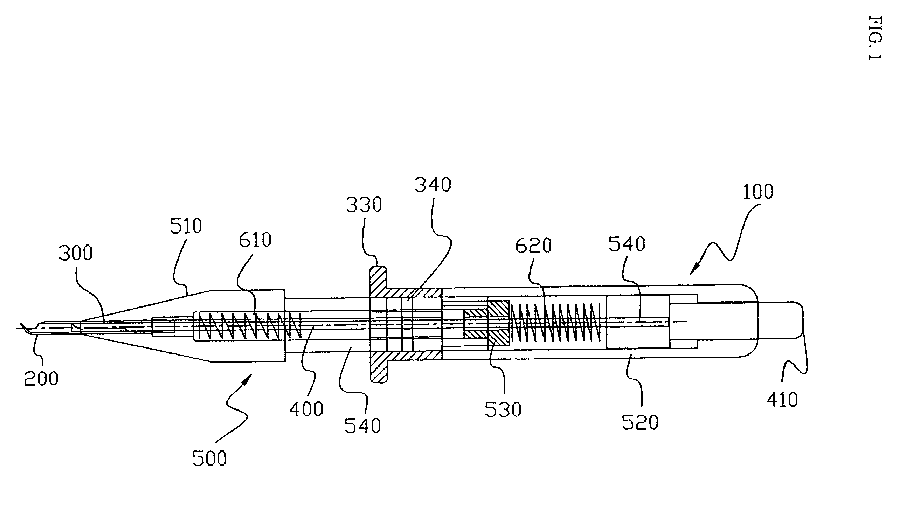

With reference to FIG. 1 showing a first preferred embodiment according the present invention, hair transplant device 100 comprises cylindrical first cutting member 200, cylindrical second cutting member 300, extrusion member 400, first operating handle 330 controlling movement of second cutting member 300, and second operating handle 410 controlling movement of extrusion member 400, and these modules are protected by housing 500.

Housing 500 comprises conical front part 510 and cylindrical body 520, and first cutting member 200 is affixed in conical front part 510. First operating handle 330 is connected to second cutting member 300 via perpendicular connecting member 340. First spring 610 (for simplicity, a portion thereof is emitted) is installed in conical front part 510, more specifically, between an inner portion of conical front part 510 and perpendicular connecting member 340. Second cutting member 300 moves forward/backward along an inner surface of cylindrical first cutting member 200. Extrusion member 400 moves forward/backward along an inner surface of cylindrical second cutting member 300. Induction member 530, inducing forward/backward movement of extrusion member 300, is disposed in body 520. Second spring 620 is installed between induction member 530 and bottom part 540 of body 520. First operating handle 330 moves forward/backward along induction road 540 formed on the side of body 510. On induction road 540, there is an induction flute (not shown in FIG. 1) through which perpendicular connecting part 340, connecting second cutting member 300 and first operating handle 330, moves forward/backward.

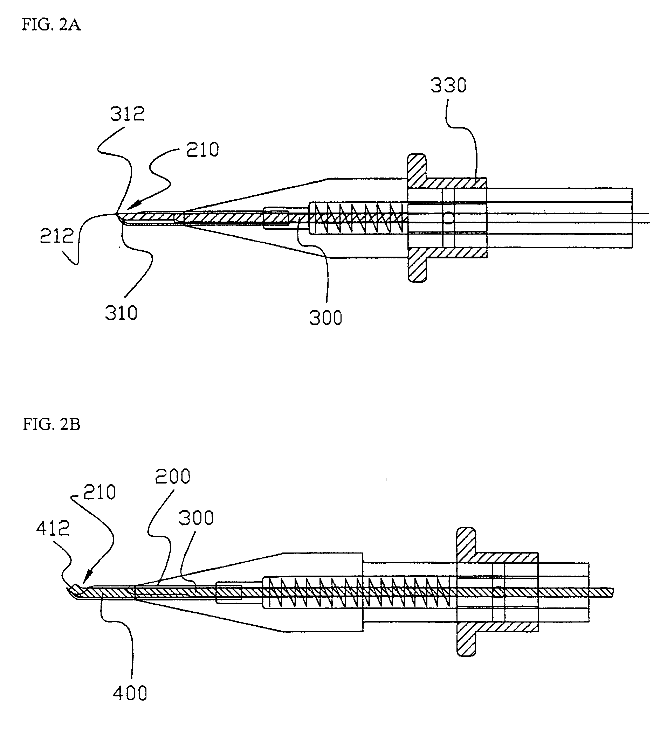

FIG. 1 shows the rest mode in which both second cutting member 200 and extrusion member 300 recede fully. FIG. 2A shows the hair-harvesting mode in which only second cutting member 200 moves forward, and FIG. 2B shows the hair-implanting mode in which only extrusion member 300 moves forward. The hair-harvesting mode in FIG. 2A is executed by pushing first operating handle 330 forward, the hair-implanting mode in FIG. 2B is executed by pressing second operating handle 410 (in FIG. 1).

Referring to FIG. 2A, while second cutting member 300 moves forward along the inner surface of first cutting member 200, blade surface 310 of second cutting member 300 cuts the bottom of a hair graft (not shown in FIGS.) having entered through entrance 210 of first cutting member 200. Forward movement of second cutting member 300 continues, until distal end 312 thereof meets distal end 212 of first cutting member 200, wherein the distance of movement of second cutting member 300 is the same as that of movement of first operating handle 330.

Referring to FIG. 2B, second cutting member 300 has receded, when extrusion member 400 being flexible arrives at a front portion of first cutting member 200 after forward movement along an inner surface of second cutting member 300. Extrusion member 400 pushes out the hair graft (not shown in FIGS.), while it moves along an inner surface of the curved front portion of first cutting member 200. In FIG. 2B, distal end 412 of extrusion member 400 protrudes at little from entrance 210 of first cutting member 400 because extrusion member 400 is made of a flexible material. However, distal end 412 made of a rigid material does not so, which will not hinder extrusion of a hair graft from entrance 210 of first cutting member 200.

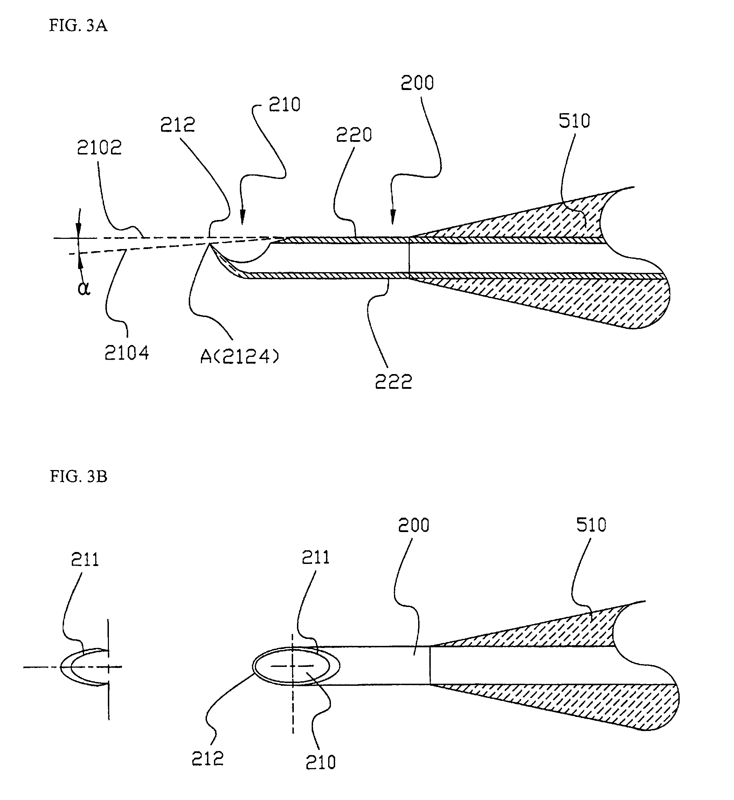

With reference to FIGS. 3A and 3B, the front portion of first cutting member 200 according to a preferred embodiment of the present invention is described below in detail.

In connection with entrance 210 of first cutting member 200, blade 211 has been formed on a surrounding thereof. Accordingly, as first cutting member 200 is inserted into a scalp, blade 211 cuts the scalp to form a hair graft. At the sight of a perpendicular cross section, lower side 222 is gently bent toward entrance 210. A summit of such curve forms distal end 212. Imaginary surface 2102 of entrance 210 is generally parallel to upper side 220; however, in any case, it may be inclined a little from upper side 220 as in FIG. 3A. In this case, distal end 212 is at the position "A(2124)", and the distal end (310 shown in FIGS. 2A and 2B) of the second cutting member (300 shown in FIGS. 2A and 2B) is also bent such that distal end 310 meets distal end 2124 at fully forward movement of the second cutting member. A harvest amount of a hair graft is dependent upon the area and angle of entrance 210 of first cutting member 200. That is, the more the area of entrance 210 is, the more the harvest amount is. Also, the more the angle "α" of entrance 210 is, the less the harvest amount is. The cross-sectional shape of entrance 210 is concave in FIG. 3A, but it may be flat.

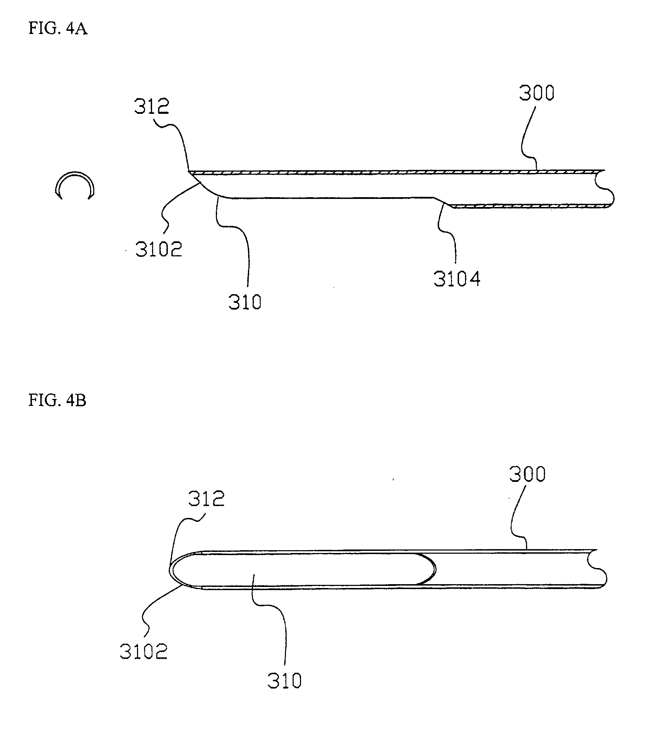

With reference to FIGS. 4A and 4B, the front portion of second cutting member 300 according to a preferred embodiment of the present invention is described below in detail.

The direction of blade 310 of second cutting member 300 is approximately opposite to entrance (210 shown in FIGS. 3A and 3B) of first cutting member (200 shown in FIGS. 3A and 3B), which is for cutting the bottom of a hair graft, introduced through entrance 210 of first cutting member 200, with blade 310 of second cutting member 300. Distal end 312 of second cutting member 300 should meet the distal end (212 shown in FIGS. 3A and 3B) of first cutting member 200 for cutting of the hair graft. Therefore, as described above, in the case that distal end 210 of first cutting member 200 inclines a little, distal end 312 of second cutting member 312 should be also bent to the extent corresponding thereto.

Meanwhile, since blade 310 of second cutting member 300 touches the inner surface of the curved front portion of first cutting member 200, it is preferably made in the form of curve 3102 as seen in FIG. 4A. To minimize a damage of a hair graft at cutting, blade surface 310 has sunken structure 3104.

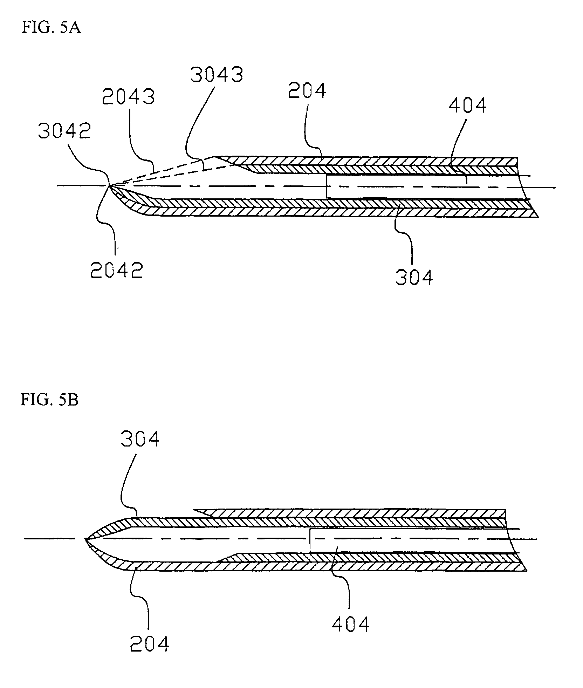

With reference to FIGS. 5A and 5B, modified first cutting member 204 and second cutting member 304 according to a preferred embodiment of the present invention is described below in detail. A feature of these member 204, 304 is that second cutting member 304 is operated in not forward/backward movement type but revolving type, and that the shape of second cutting member 304 is the same as that of first cutting member 204. Extrusion member 404 is the same as that in the forward/backward movement type.

Distal end 2042 of first cutting member 204 is bent to a central axis thereof, and distal end 3042 of second cutting member 304 is also bent to the central axis. At a rest mode (FIG. 5A), when entrance 3043 of second cutting member 304 is in the same position as that of entrance 2043 of first cutting member 204, entrance 2043 of first cutting member 204 becomes opened. To the contrary, as second cutting member 304 revolves, its blade cuts the bottom of a hair graft (not shown in FIGS.). Revolution of second cutting member 304 can be accomplished with a handle type based on the manual manner or a switch type based on the automatic manner, and configurations for them can be easily assumed from the above description by the those of ordinary skill in the art. As such, the detailed description thereof is not provided.

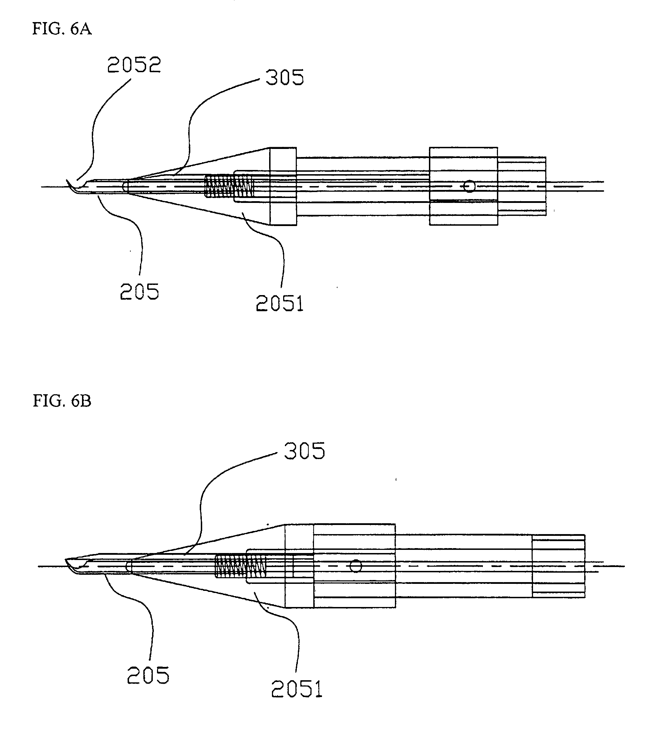

With reference to FIGS. 6A and 6B, modified first cutting member 205 and second cutting member 305, according to a preferred embodiment of the present invention, are described below in detail. A feature of this modified configuration is that second cutting member 305 moves forward and backward along the outer surface of first cutting member 205. First cutting member 205 is affixed in conical front part 2051, and second cutting member 305 moves forward/backward along the outer surface of first cutting member 205. Second cutting member 305 is not made in a cylindrical form as in other embodiments, but is made in a warped plate or half-cylindrical form for the purpose of shutting at least entrance 2052 of first cutting member 205. If second cutting member 305 is made in a cylindrical form, first cutting member 205 to be disposed inside second cutting member 305 cannot be affixed in conical front part 2051

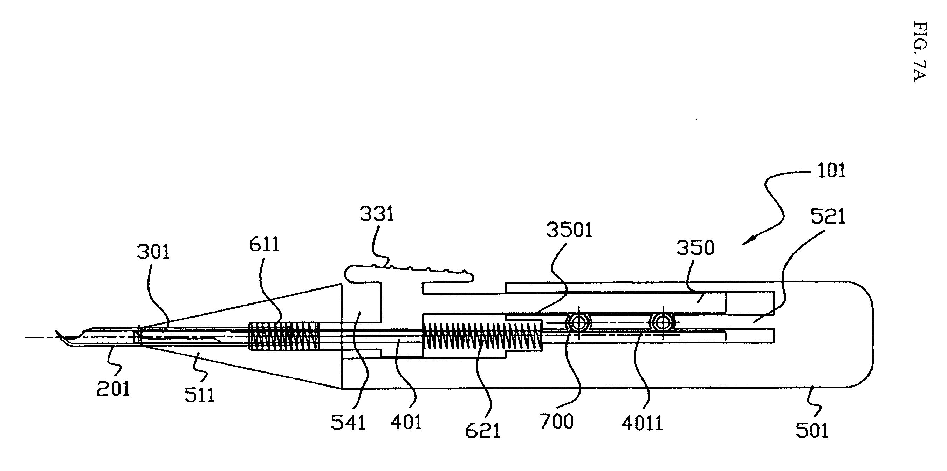

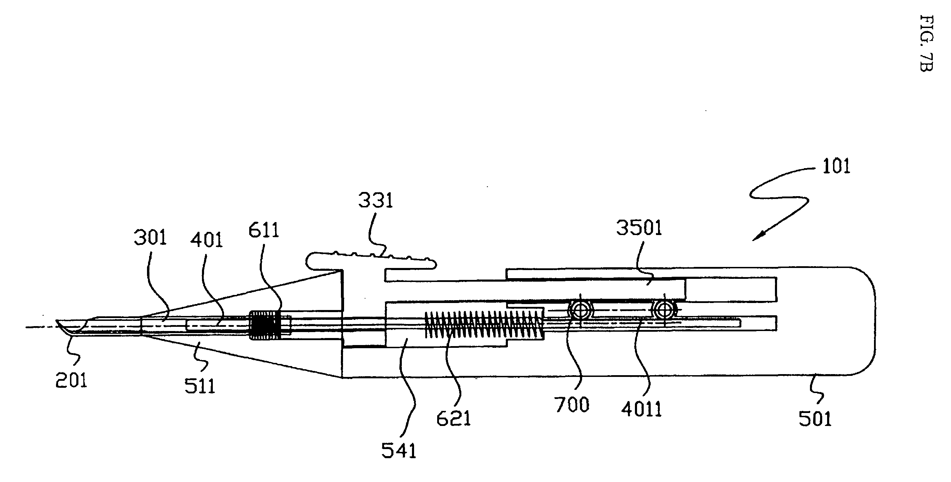

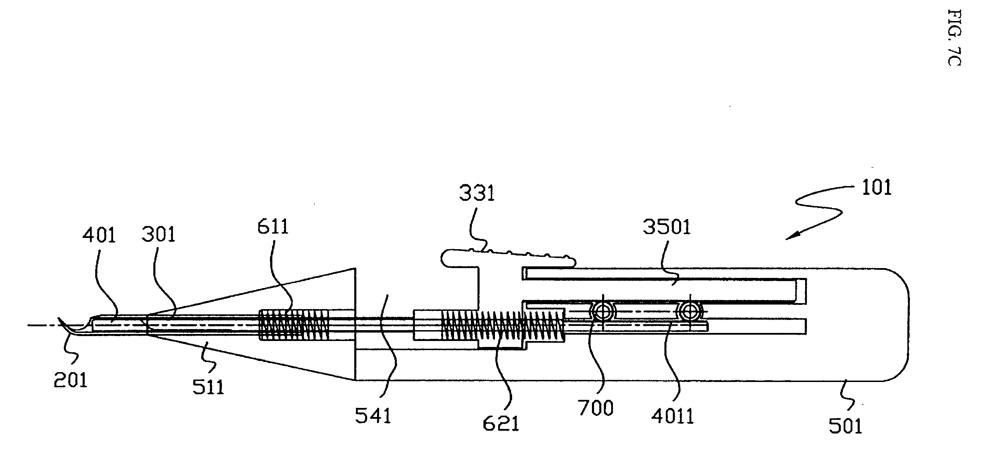

With reference to FIGS. 7A - 7C, a hair transplant device 101 according to a second embodiment of the present invention is described below in detail.

First cutting member 201 and second cutting member 301 in hair transplant device 101 is identical with those in hair transplant device 100 (shown in FIG. 1), and it is different in that a forward/backward movement of second cutting member 301 and extrusion member 401 is controlled by only one operating handle 331. On the interior of body 521 of housing 501, gear 700 for reversing the direction of movement is installed with its axis affixed on body 521. In addition, threads 3501, 4011 are formed on a bottom surface of rear extension part 350 of operating handle 331 and a top surface of extrusion member 401, respectively. Operating handle 331 is connected to second cutting member 301. Therefore, when second cutting member 301 is pushed forward, rear extension part 350 also moves forward and thread 3501 of rear extension part 350 shifts left, thereby forcing gear 700 to revolve counterclockwise. At the same time, thread 4011 of extrusion member 401 shifts right, and thus extrusion member 401 moves backward. Forward movement of extrusion member 401, resulted from backward movement of second cutting member 301, is executed in the manner contrary to the above.

First spring 611 affixed in conical front part 511 provides operating handle 331 with a recovering force at forward movement of the latter, and second spring 621 installed between operating handle 331 and body 521 provides operating handle 331 with a recovering force at backward movement of the latter. Accordingly, when no force is applied, operating handle 331 is automatically positioned in the middle of induction road 541.

FIG. 7A shows hair transplant device 101 under a rest mode, FIG. 7B shows one under a hair-harvesting mode, and FIG. 7C shows one under a hair-implanting mode. Operating handle 331 is at the middle position of induction road 541 under the rest mode, at the front position under hair-harvesting mode, and at the rear position under hair-implanting mode, respectively.

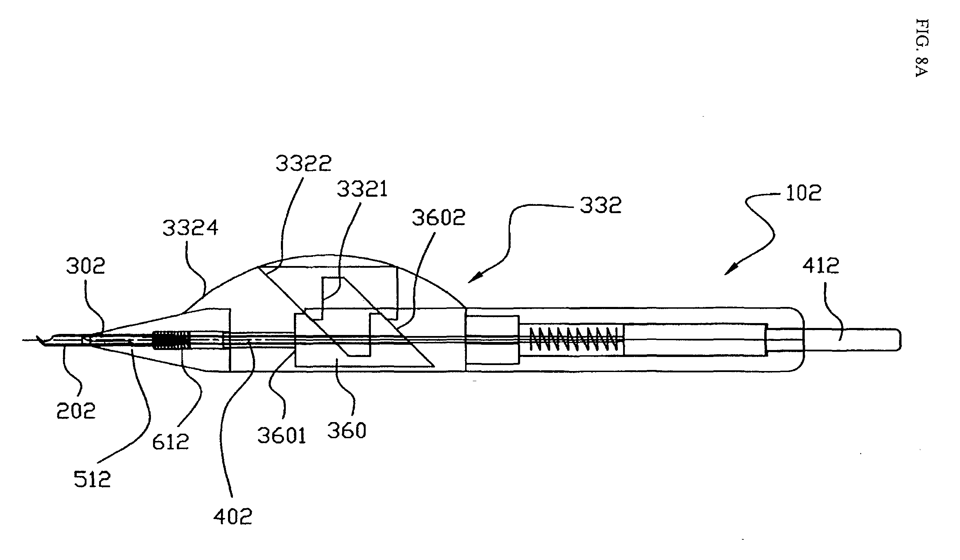

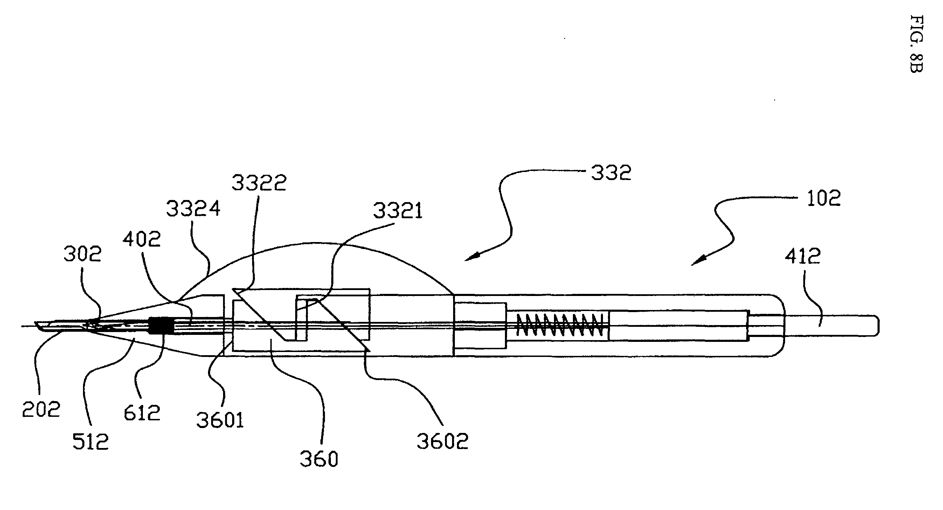

With reference to FIGS. 8A - 8C, hair transplant device 102 according to a third preferred embodiment of the present invention is described below in detail.

First cutting member 202, second cutting member 302, extrusion member 402, and second operating handle 412 are the same as those in hair transplant device 100 (shown in FIG. 1), but it is different in that a configuration for operating second cutting member 302 is a button type. More specifically, first triangle member 360 is installed on second cutting member 302, wherein perpendicular surface 3601 of first triangle member 360 is disposed forward. In addition, second triangle member 332 is installed in first operating handle 332 for operating second cutting member 302, wherein slop surface 3322 of second triangle member 332 is in contact with slop surface 3602 of first triangle member 360, and exterior member 3342 laps them. Therefore, when second triangle member 332 is pushed down, its slope surface 3322 presses corresponding slop surface 3602 of first right triangle member 360. To remove the pressure, first triangle member 360 shifts left, and thus second cutting member 302 connected thereto moves forward. If the pressure applied to second triangle member 332 is removed, then second cutting member 302 will move backward by first spring 612 installed in conical front part 512 and, at this time, second triangle member 332 will rise.

FIG. 8A shows hair transplant device 102 under a rest mode, FIG. 8B shows one under a hair-harvesting mode, and FIG. 8C shows one under a hair-implanting mode, respectively.

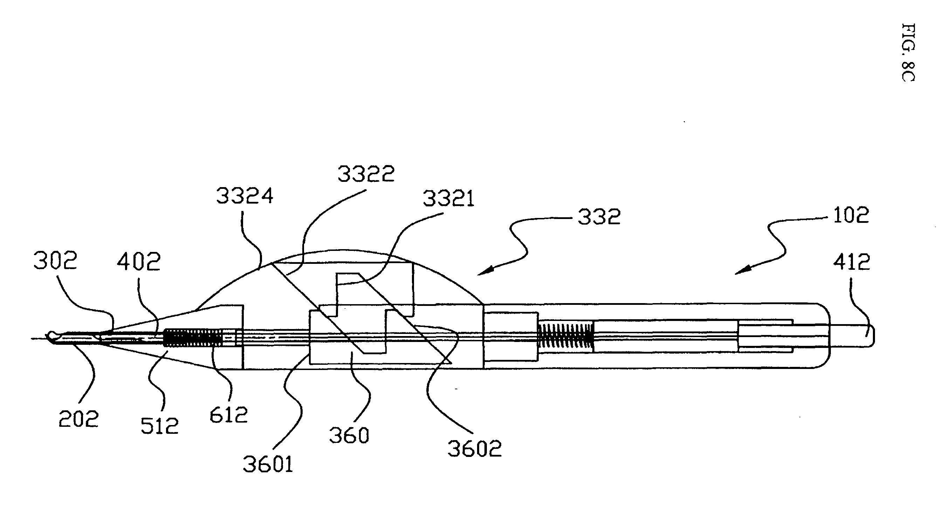

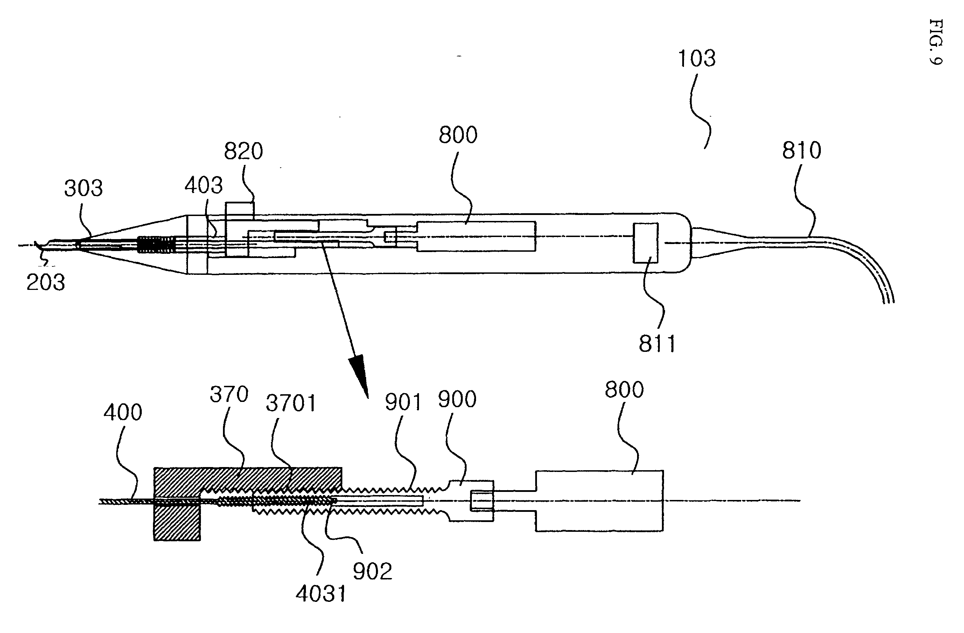

With reference to FIG. 9, hair transplant device 103 according to a fourth preferred embodiment of the present invention is described below in detail.

Regarding automatic type-hair transplant device 103, first cutting member 203, second cutting member 303 and extrusion member 403 are the same as those in manual type-hair transplant devices 100, 101, 102, but it is different from these devices in that a power source is electricity for operating second cutting member 303 and extrusion member 403.

Extension part 370 which has thread 3701 on an inner surface thereof is connected to the rear of second cutting member 303. Thread 4031 is formed on an outer surface of the rear of extrusion member 403. The directions of the two threads 3701, 4031 are opposite to each other. Power-transmitting member 900, which works as transmitting a revolution of electric motor 800 to extension part 370 and extrusion member 403, is made in a cylindrical form at the portion engaged with extension part 370 and extrusion member 403. Herein, thread 901 corresponding to thread 3701 of extension part 370 is formed on the outer surface of cylindrical power-transmitting member 900, and thread 902 corresponding to thread 4031 of extrusion member 403 is formed on the inner surface thereof.

Therefore, for example, when electric motor 800 revolves clockwise, power-transmitting member 900 connected thereto also revolves clockwise, and thereby extension part 370 moves forward during revolution of thread 901, and at the same time extrusion member 403 moves backward during revolution of thread 902. Backward movement of extension part 370 and forward movement of extrusion member 403 are carried out by counterclockwise revolution of electric motor 800 in the opposite manner to the above.

Power of electric motor 800 is supplied from the exterior via cable 810; however, in any case, a power supply may be first battery or secondary rechargeable battery installed in housing 503. Switch 820 for controlling (clockwise and counterclockwise) operation of electric motor 800 is installed on housing 503; however, in any case, the switch may be installed exterior to housing 503. For example, such exterior switch may be operated with feet or hands of a surgeon.

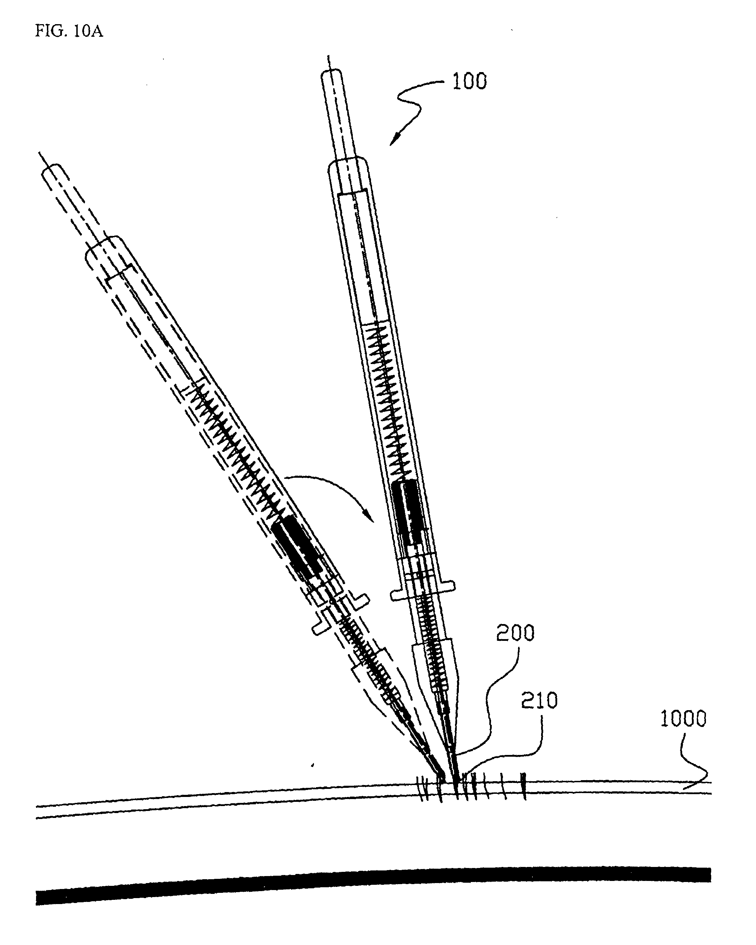

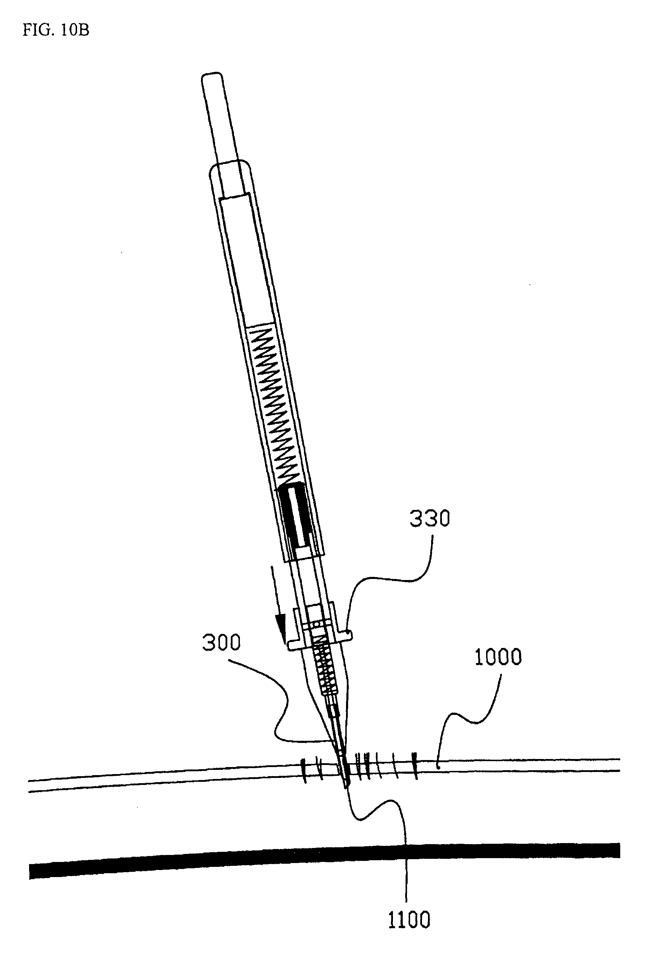

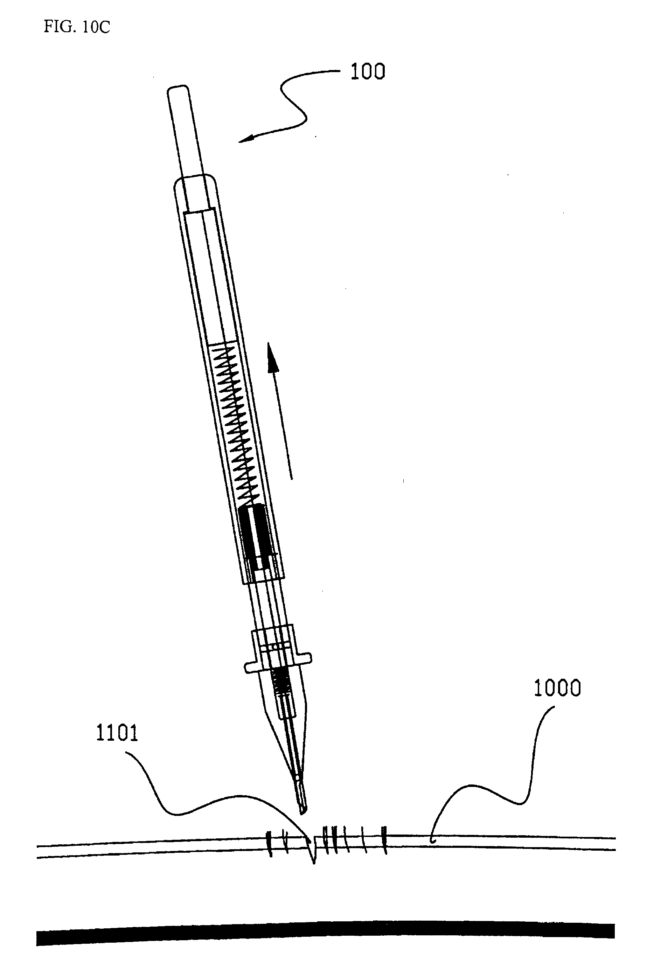

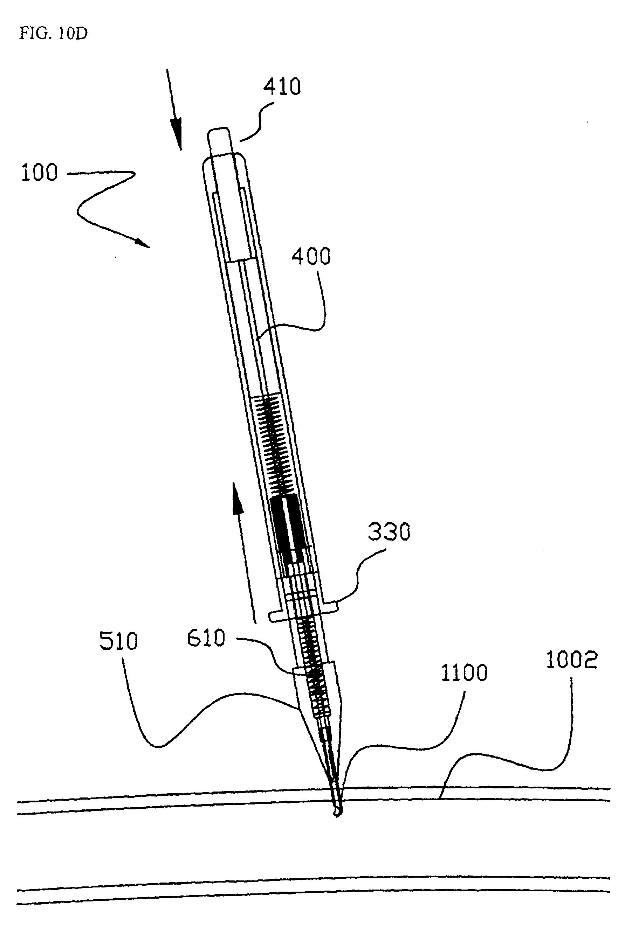

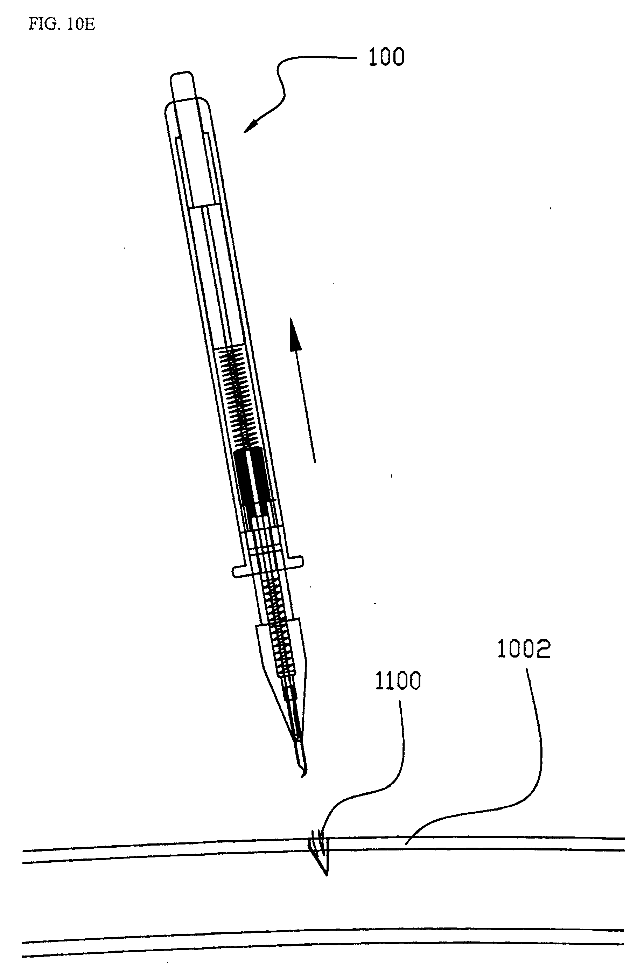

With reference to FIGS. 10A - 10E, the serial transplantation procedure of harvesting a hair graft from a hairy area in a scalp and then implanting the hair graft into a bald area is described below in detail.

Referring to FIG. 10A, hair transplant device 100 under a rest mode is inserted into hairy area 1000 for harvesting a hair graft. In the rest mode, since second cutting member 200 recedes, entrance 210 of first cutting member 100 is open. In order that a blade of first cutting member 100 cuts a skin during insertion of first cutting member 200, hair transplant device 100 is inserted as moving from a rather inclined state with entrance 210 facing the skin to a perpendicular state. This may be compared to inserting a scoop into a lump of ice cream for the purpose of scooping ice cream.

Next, referring to FIG. 10B, first operating handle 330 is pushed forward and, thereby, second cutting member 300 cuts the bottom of hair graft 1100 having been introduced into device 100.

Referring to FIG. 10C, the hair graft-harvesting step is finished by withdrawing hair transplant device 100 from hairy area 1000 with maintaining the state that first operating handle 330 is pushed forward. After removal of the hair graft, hole 1101 is left on hairy graft, which is very small and thus can be speedily healed without leaving a scar.

The hair graft-implanting step is started from insertion of hair transplant device 100 into a bald area 1002. Referring to FIG. 10D, hair graft 1100 harvested has been safely loaded in hair transplant device 100 under the state that first operating handle 330 has been pushed forward. Hair transplant device 100 under such a state is inserted into a bald area 1001 of a scalp. At this time, the direction of insertion is almost perpendicular to the scalp, which is different from that in the hair graft-harvesting step. The entrance of the first cutting member, shut by the second cutting member, can be easily inserted into the scalp. After completion of insertion, when a forward pressure applied to first operating handle 330 is removed, the second cutting member moves backward by a recovering force of first spring 610 and, thereby, the entrance of first cutting member becomes opened. After that, as second operating handle 410 is pressed, hair graft 1100 loaded in hair transplant device 100 is pushed out by forward movement of extrusion member 400.

Next, referring to FIG. 10E, hair transplant device 100 is withdrawn from the scalp, and then hair graft 1100 is implanted into bald area 1002. As a result, the hair-transplanting procedure is completed.

### INDUSTRIAL APPLICABILITY

According to hair transplant devices of the present invention, it is possible to execute a hair-transplanting procedure, i.e., harvesting a hair graft from a hairy area in a scalp and then implanting the hair graft into a bald area thereof, in series without using other devices. Also, it is not necessary to incise rather big size of the scalp for harvest of hair grafts, and only very small size of a perforation is required. Therefore, no scars are left on the perforated site of the hairy area. Furthermore, since only the portion necessary for transplantation is easily harvested, the scalp is not injured. It is not required to perforate on the bald area for implantation, and thus a patient does not feel a pain. The possibility of depilation decreases remarkably, and hairs after implantation are engrafted very well, because the bald area is spread without perforation and then the hair graft is implanted therein.

The present invention has been described with to particular illustrative embodiments. It is to be understood that the invention is not limited to the above-described embodiments and modifications thereto, and the various changes and modifications may be made by those of ordinary skill in the art without departing from the scope of the appended claims. For example, modified devices, which has constitutional elements as described above, and of which some elements are shortened with respect to the size or changed with respect to the ratio of size, may be useful as devices for harvesting tissues from human body for a histo-biopsy. That is, these devices can be used to harvest tissues from human organ such as liver, pancreas, breast, thyroid gland, kidney, and so on for the purpose of testing various diseases.

## Claims

1. A device for executing a hair-transplanting procedure, of harvesting a hair graft from a hairy area in a scalp and then implanting the hair graft into a bald area, in series, comprising:
first cutting member (200-205) affixed to a front part of the present device and having an elongate and hollow shape, wherein the first cutting member has a blade (211) and the scalp is cut by the blade and an extrusion member (400-403)
having an elongate shape, wherein the extrusion member pushes out a hair graft loaded in the present device through an entrance (210) of the blade surface (211) of the first cutting member, as the extrusion member moves forward;
**characterized by** a surface of the blade (211) that inclines toward a side of the first cutting member; by the fact that the hair graft comes from the cutting of the scalp and is then introduced into the first cutting member; by a
second cutting member (300-305) disposed to move forward/backward (or revolve) in the first cutting member and having an elongate and hollow shape, wherein the second cutting member has a blade (310) of which the surface cuts a bottom portion of the hair graft having been introduced into the present device and then meets the blade surface of the first cutting member, as the second cutting member moves forward; by the fact that the extrusion member is disposed to move forward/backward in the second cutting member and by a
housing (500-503) forming an outward shape and providing an induction road for movements of the second cutting member and the extrusion member.

2. The device according to claim 1, wherein the first cutting member (200-205) is made in a cylindrical form, and the blade surface (211) which forms the entrance of the present member inclines toward the side of the cylindrical member, and the opposite side of the cylindrical member is gently bent to be extended toward the above side (which the blade surface faces), and a cross-sectional shape of the blade surface is ellipse or circle; and
wherein the second cutting member (300-305) is made in a cylindrical form, and the direction of the blade surface of the cylindrical member is approximately opposite to the direction of the blade surface of the first cutting member.

3. The device according to claim 1, wherein the axis of the blade surface (211) of the first cutting member (200-205) inclines a little, such that this blade surface is seen a little from the front sight of the present device; the second cutting member parts from an imaginary extended line of the side of the first cutting member, to the extent that the end of the first cutting member parts from the imaginary extended line of the side thereof, whereby the end of the first cutting member can ultimately meet the end of the second cutting member (300-305) when the latter moves forward.

4. The device according to claim 1, the first cutting member (200-205) is made in a cylindrical form, and an end of the first cutting member is bent to an central axis thereof; and the second cutting member (300-305), disposed in the first cutting member, has the same shape as the first cutting member, whereby an entrance (210) of the first cutting member becomes opened when each blade surface of both members is in the same position, and the entrance becomes closed as the second cutting member revolves.

5. The device according to claim 1, the extrusion member is made of flexible materials, and an end thereof protrudes a little from an entrance of the first cutting member when it moves forward fully.

6. The device according to one of claims 1 through 5, wherein
the first cutting member is affixed in the conical front part of the housing;
the second cutting member is supported by a first spring (610-611-612) installed in the conical front part, and is connected to a first operating handle (330-332) disposed at the middle of a body of the housing; and
the extrusion member is supported by a second spring (620-621) installed in the body, and is connected to a second operating handle (410-412) disposed at the rear of the body.

7. The device according to one of claims 1 through 5, wherein
the first cutting member is affixed in the conical front part of the housing,
the second cutting member is supported by a first spring (610-611-612) installed in the conical front part, and is connected to an operating handle (331) disposed at the middle of a body (521) of the housing, wherein a rear portion of the operating handle is extended to a power transmitting member (900);
the extrusion member is supported by a second spring (620-621) installed between a connecting axis to the operating handle and a middle portion of the body, and is extended to the power transmitting member; and
the power transmitting member comprises a gear (700) which revolves with its axis affixed in the body, wherein the gear engages with a thread (3501) which is formed on the bottom of a rear extension part (350) of the operating handle (331) and a thread (4011) which is formed on a top of a rear extension part of the extrusion member, (401) respectively.

8. The device according to one of claims 1 through 5, wherein
the first cutting member is affixed in the conical front part;
the second cutting member is supported by a first spring (610-611-612) installed in the conical front part, wherein triangle members (360) are installed on the second cutting member (302) at the position corresponding to a first operating handle (332) disposed on the middle of a body of the housing, wherein a perpendicular face (3601) of the first triangle is arranged forwardly;
the extrusion member is supported by a second spring (620-621) installed in the middle of the body, and is connected to a second operating handle (412) disposed at the rear of the body; and
the first operating handle includes second triangle members, (332) wherein a slope face (3322) of the second triangle member is sliding contact with a slope face (3602) of the first triangle member.

9. The device according to one of claims 1 through 5, wherein
the first cutting member is affixed in the conical front part;
the second cutting member includes an extension part (370) at the rear thereof, wherein an inner surface of the extension part has a thread (3701) engaged with a power-transmitting member linked to an electric motor; (800)
the extrusion member (403) includes a thread (4031) engaged with the power-transmitting member on an outer surface thereof; and
the power-transmitting member includes a thread (901) engaged with the thread (3701) of the extension part, and a thread (902) engaged with the thread (4031) of the extrusion member, wherein the two threads of the power-transmitting member are formed at the opposite direction to each other.

## Patentansprüche

1. Vorrichtung zur Ausführung einer Haartransplantationsprozedur, nacheinander ein Haartransplantat von einem behaarten Bereich auf der Kopfhaut zu ernten und dann das Haartransplantat in einem kahlen Bereich zu implantieren, umfassend:
ein erstes schneidendes Element (200 - 205), an einem vorderen Abschnitt der vorliegenden Vorrichtung angebracht und eine längliche, hohle Gestalt besitzend, wobei das erste schneidende Element eine Klinge (211) besitzt und die Kopfhaut mit der Klinge geschnitten wird, und ein Extrusionselement (400 - 403), eine längliche Gestalt besitzend, wobei das Extrusionselement bei seiner Vorwärtsbewegung ein durch eine Eintrittsöffnung (210) der Klingenoberfläche (211) des ersten schneidenden Elements in die vorliegende Vorrichtung aufgenommenes Haartransplantat herausdrückt;
**gekennzeichnet durch** eine Oberfläche der Klinge (211), die sich einer Seite des ersten schneidenden Elements zuneigt; **dadurch**, dass das Haarimplantat vom Schneiden der Kopfhaut kommt und dann in das erste schneidende Element eingeführt wird; **durch** ein zweites schneidendes Element (300 - 305), so angeordnet, dass es sich im ersten schneidenden Element vorwärts/rückwärts bewegt (oder dreht) und eine längliche, hohle Gestalt besitzt, wobei das zweite schneidende Element eine Klinge (310) besitzt, deren Oberfläche einen unteren Abschnitt des Haartransplantats abschneidet, das in die vorliegende Vorrichtung eingeführt worden ist, und bei seiner Vorwärtsbewegung dann auf die Klingenoberfläche des ersten schneidenden Elements trifft; **dadurch**, dass das Extrusionselement so angeordnet ist, dass es sich im zweiten schneidenden Element vorwärts/rückwärts bewegt; und **durch** ein Gehäuse (500 - 503), das eine äussere Gestalt bildet und einen Induktionsweg für die Bewegungen des zweiten schneidenden Elements und des Extrusionselements bietet.

2. Vorrichtung nach Anspruch 1, **dadurch gekennzeichnet, dass** das erste schneidende Element (200 - 205) zylindrisch ausgebildet ist und die Klingenoberfläche (211), die die Eingangsöffnung dieses Elements bildet, sich der Seite des zylindrischen Elements zuneigt, während die entgegengesetzte Seite des zylindrischen Elements gelinde gebogen ist, um sich zu der obigen Seite hin zu erstrecken (die der Klingenoberfläche gegenüber steht), und ein Querschnitt der Klingenoberfläche eine Ellipse oder ein Kreis ist; und
**dadurch**, dass das zweite schneidende Element (300 - 305) zylindrisch ausgebildet ist und die Richtung der Klingenoberfläche des zylindrischen Elements zur Richtung der Klingenoberfläche des ersten schneidenden Elements ungefähr entgegengesetzt ist.

3. Vorrichtung nach Anspruch 1, **dadurch gekennzeichnet, dass** die Achse der Klingenoberfläche (211) des ersten schneidenden Elements (200 - 205) ein wenig geneigt ist, so dass diese Klingenoberfläche von der Vordersicht der vorliegenden Vorrichtung her ein wenig zu sehen ist; das zweite schneidende Element sich von einer gedachten verlängerten Linie der Seite des ersten schneidenden Elements weg erstreckt, und zwar so, dass sich das Ende des ersten schneidenden Elements von der gedachten verlängerten Linie seiner Seite weg erstreckt, wodurch das Ende des ersten schneidenden Elements letztlich auf das Ende des zweiten schneidenden Elements (300 - 305) treffen kann, wenn letzteres sich nach vom bewegt.

4. Vorrichtung nach Anspruch 1, das erste schneidende Element (200 - 205) ist zylindrisch ausgebildet, und ein Ende des ersten schneidenden Elements ist zu einer zentralen Achse davon hingebogen; und das zweite schneidende Element (300 - 305), im ersten schneidenden Element angeordnet, hat die gleiche Gestalt wie das erste schneidende Element, wodurch eine Eingangsöffnung (210) des ersten schneidenden Elements geöffnet wird, wenn sich die Klingenoberflächen der beiden Elemente in der gleichen Position befinden, aber die Eingangsöffnung geschlossen wird, wenn sich das zweite schneidende Element dreht.

5. Vorrichtung nach Anspruch 1, das Extrusionsglied besteht aus biegsamen Materialien, und ein Ende davon ragt ein wenig aus einer Eingangsöffnung des ersten schneidenden Elements hervor, wenn es sich ganz nach vom bewegt.

6. Vorrichtung nach einem der Ansprüche 1 bis 5, **dadurch gekennzeichnet, dass** das erste schneidende Element im konischen vorderen Abschnitt des Gehäuses angebracht ist; das zweite schneidende Element von einer ersten Feder (610, 611, 612) gehalten wird, die im konischen vorderen Abschnitt eingebaut ist, und mit einem ersten Bedienungsgriff (330 - 332) verbunden ist, der in der Mitte des Gehäusekörpers angeordnet ist; und
das Extrusionselement von einer zweiten Feder (620 - 621) gehalten wird, die im Körper eingebaut ist, und mit einem zweiten Bedienungsgriff (410 - 412) verbunden ist, der hinten am Körper angeordnet ist.

7. Vorrichtung nach einem der Ansprüche 1 bis 5, **dadurch gekennzeichnet, dass** das erste schneidende Element im konischen vorderen Abschnitt des Gehäuses angebracht ist, das zweite schneidende Element von einer ersten Feder (510, 611, 612) gehalten wird, die im konischen vorderen Abschnitt eingebaut ist, und mit einem Bedienungsgriff (331) verbunden ist, der in der Mitte eines Gehäusekörpers (521) angeordnet ist, wobei ein hinterer Abschnitt des Bedienungsgriffs zu einem kraftübertragenden Element (900) hin verlängert ist;
das Extrusionselement von einer zweiten Feder (620 - 621) gehalten wird, die zwischen einer Verbindungsachse zum Bedienungsgriff und einem mittleren Abschnitt des Körpers eingebaut ist, und zum kraftübertragenden Element hin verlängert ist; und
das kraftübertragende Element ein Zahnrad (700) umfasst, das sich um seine im Körper angebrachte Achse dreht, wobei das Zahnrad in ein Gewinde (3501), das am Boden eines rückwärtigen Verlängerungsabschnitts (350) des Bedienungsgriffs (331) ausgebildet ist, sowie in ein Gewinde (4011), das an der Oberseite eines rückwärtigen Verlängerungsabschnitts des Extrusionselements (401) ausgebildet ist, eingreift.

8. Vorrichtung nach einem der Ansprüche 1 bis 5, **dadurch gekennzeichnet, dass**
das erste schneidende Element im konischen vorderen Abschnitt angebracht ist;
das zweite schneidende Element durch eine erste Feder (610, 611, 612) gehalten wird, die im konischen vorderen Abschnitt eingebaut ist, wobei dreieckige Elemente (360) auf dem zweiten schneidenden Element (302) an der Stelle aufgesetzt sind, die einem ersten Bedienungsgriff (332) entspricht, der in der Mitte eines Gehäusekörpers angeordnet ist, wobei eine senkrechte Seite (3601) des ersten Dreiecks nach vom hin angeordnet ist;
das Extrusionselement durch eine zweite Feder (620 - 621) gehalten wird, die in der Mitte des Körpers eingebaut ist, und mit einem zweiten Bedienungsgriff (412) verbunden ist, der hinten am Körper angeordnet ist; und
der erste Bedienungsgriff zweite dreieckige Elemente (332) umfasst, wobei eine geneigte Seite (3322) des zweiten dreieckigen Elements in gleitender Berührung mit einer geneigten Seite (3602) des ersten dreieckigen Elements steht.

9. Vorrichtung nach einem der Ansprüche 1 bis 5, **dadurch gekennzeichnet, dass**
das erste schneidende Element im konischen vorderen Abschnitt angebracht ist;
das zweite schneidende Element an seiner hinteren Seite einen verlängerten Abschnitt (370) einschliesst, wobei eine Innenseite des verlängerten Abschnitts ein Gewinde (3701) besitzt, das in ein kraftübertragendes Element eingreift, das mit einem Elektromotor (800) verbunden ist;
das Extrusionselement (403) ein Gewinde (4031) besitzt, das in das kraftübertragende Element an dessen Aussenseite eingreift; und
das kraftübertragende Element ein Gewinde (901) einschliesst, das in das Gewinde (3701) des verlängerten Abschnitts eingreift, sowie ein Gewinde (902), das in das Gewinde (4031) des Extrusionselements eingreift, wobei die beiden Gewinde des kraftübertragenden Elements in zueinander entgegengesetzten Richtungen ausgebildet sind.

## Revendications

1. Dispositif servant à exécuter une procédure de transplantation de cheveux, consistant à prélever une greffe de cheveu dans une zone chevelue d'un cuir chevelu et ensuite à implanter la greffe de cheveu dans une zone chauve, successivement, comprenant :
un premier élément coupant (200 - 205) fixé sur une partie avant du présent dispositif et possédant une forme allongée et creuse, dans lequel le premier élément coupant possède une lame (211) et le cuir chevelu est coupé par la lame et un élément d'expulsion (400 - 403) possédant une forme allongée, dans lequel l'élément d'expulsion pousse vers l'extérieur une greffe de cheveu chargée dans le présent dispositif à travers une entrée (210) de la surface de la lame (211) du premier élément coupant, à mesure que l'élément d'expulsion se déplace vers l'avant ;
**caractérisé par** une surface de la lame (211) qui s'incline en direction d'un côté du premier élément coupant ; par le fait que la greffe de cheveu provient de la coupe du cuir chevelu et est ensuite introduite dans le premier élément coupant ; par
un second élément coupant (300 - 305) disposé pour se déplacer vers l'avant/vers l'arrière (ou pour tourner) dans le premier élément coupant et possédant une forme allongée et creuse, dans lequel le second élément coupant possède une lame (310) dont la surface coupe une portion inférieure de la greffe de cheveu qui a été introduite dans le présent dispositif et rencontre ensuite la surface de la lame du premier élément coupant à mesure que le second élément coupant se déplace vers l'avant ; par le fait que l'élément d'expulsion est disposé de façon à se déplacer vers l'avant/vers l'arrière dans le second élément coupant et par
un logement (500 - 503) formant une forme vers l'extérieur et fournissant une voie d'induction pour les mouvements du second élément coupant et de l'élément d'expulsion.

2. Dispositif selon la revendication 1, dans lequel le premier élément coupant (200 - 205) est réalisé de forme cylindrique, et la surface de la lame (211) qui forme l'entrée du présent élément s'incline en direction du côté de l'élément cylindrique, et le côté opposé de l'élément cylindrique est doucement recourbé pour s'étendre en direction du côté ci-dessus (auquel la surface de la lame fait face) et la forme en coupe de la surface de la lame est elliptique ou circulaire ;
et
dans lequel le second élément coupant (300 - 305) est réalisé de forme cylindrique et la direction de la surface de la lame de l'élément cylindrique est approximativement face à la direction de la surface de la lame du premier élément coupant.

3. Dispositif selon la revendication 1, dans lequel l'axe de la surface de la lame (211) du premier élément coupant (200 - 205) s'incline un peu, de telle façon que cette surface de la lame est un peu visible dans une vue de face du présent dispositif ; le second élément coupant s'écarte d'une ligne imaginaire prolongée du côté du premier élément coupant dans la mesure où l'extrémité du premier élément coupant s'écarte de la ligne imaginaire prolongée du côté de celui-ci, moyennant quoi l'extrémité du premier élément coupant peut finalement rejoindre l'extrémité du second élément coupant (300 - 305) lorsque ce dernier se déplace vers l'avant.

4. Dispositif selon la revendication 1, le premier élément coupant (200 - 205) est réalisé de forme cylindrique et une extrémité du premier élément coupant est recourbée par rapport à un axe central de celui-ci ; et le second élément coupant (300 - 305), disposé dans le premier élément coupant, a la même forme que le premier élément coupant, moyennant quoi une entrée (210) du premier élément coupant s'ouvre lorsque chaque surface de lame des deux éléments est dans la même position et l'entrée se ferme lorsque le second élément coupant tourne.

5. Dispositif selon la revendication 1, l'élément d'expulsion est réalisé dans des matériaux souples et une extrémité de celui-ci dépasse un peu de l'entrée du premier élément coupant lorsqu'il se déplace complètement vers l'avant.

6. Dispositif selon une des revendications 1 à 5, dans lequel
le premier élément coupant est fixé dans la partie avant conique du logement ;
le second élément coupant est supporté par un premier ressort (610 - 611 - 612) installé dans la partie avant conique, et est connecté à une première poignée de manoeuvre (330 - 332) disposée au milieu du corps du logement ; et
l'élément d'expulsion est supporté par un second ressort (620 - 621) installé dans le corps et est connecté à une seconde poignée de manoeuvre (410 - 412) disposée à l'arrière du corps.

7. Dispositif selon une des revendications 1 à 5, dans lequel
le premier élément coupant est fixé dans la partie avant conique du logement ;
le second élément coupant est supporté par un premier ressort (610 - 611 - 612) installé dans la partie avant conique et est connecté à une poignée de manoeuvre (331) disposée au milieu du corps (521) du logement, dans lequel une portion arrière de la poignée de manoeuvre s'étend jusqu'à un élément de transmission d'énergie (900) ;
l'élément d'expulsion est supporté par un second ressort (620 - 621), installé entre un axe de raccordement à la poignée de manoeuvre et une portion intermédiaire du corps, et s'étend jusqu'à l'élément de transmission d'énergie ; et
l'élément de transmission d'énergie comprend une roue dentée (700) qui tourne, son axe étant fixé dans le corps, dans lequel la roue dentée s'engrène respectivement avec un filetage (3501) qui est formé sur le bas d'une partie d'extension arrière (350) de la poignée de manoeuvre (331) et un filetage (4011) qui est formé sur le haut d'une partie d'extension arrière de l'élément d'expulsion (401).

8. Dispositif selon l'une des revendications 1 à 5, dans lequel
le premier élément coupant est fixé dans la partie avant conique ;
le second élément coupant est supporté par un premier ressort (610 - 611 - 612) installé dans la partie avant conique, où des éléments triangulaires (360) sont installés sur le second élément coupant (302) dans la position correspondant à une première poignée de manoeuvre (332) disposée sur le milieu du corps du logement, où une face perpendiculaire (3601) du premier triangle est disposée vers l'avant ;
l'élément d'expulsion est supporté par un second ressort (620 - 621) installé au milieu du corps et est raccordé à une seconde poignée de manoeuvre (412) disposée à l'arrière du corps ; et
la première poignée de manoeuvre inclut de seconds éléments triangulaires (332), où une face inclinée (3322) du second élément triangulaire est en contact à glissement avec une face inclinée (3602) du premier élément triangulaire.

9. Dispositif selon l'une des revendications 1 à 5, dans lequel
le premier élément coupant est fixé dans la partie avant conique ;
le second élément coupant inclut une partie d'extension (370) à l'arrière de celui-ci,
où une surface intérieure de la partie d'extension possède un filetage (3701) en prise avec un élément de transmission d'énergie relié à un moteur électrique (800) ;
l'élément d'expulsion (403) inclut un filetage (4031) en prise avec l'élément de transmission d'énergie sur une surface extérieure de celui-ci ; et
l'élément de transmission d'énergie inclut un filetage (901) en prise avec le filetage (3701) de la partie d'extension, et un filetage (902) en prise avec le filetage (4031) de l'élément d'expulsion,
où les deux filetages de l'élément de transmission d'énergie sont réalisés en sens opposé l'un par rapport à l'autre.
